# EUROPÄISCHE PATENTSCHRIFT

(11) **EP 3 380 131 B1**
(45) Veröffentlichungstag und Bekanntmachung des Hinweises auf die Patenterteilung: **30.12.2020**
(21) Anmeldenummer: 16805343.7
(22) Anmeldetag: 24.11.2016
(51) Int. Cl.: A61M 1/10, A61M 1/12

(54) **IMPLANTIERBARES FLUIDPUMPENSYSTEM**
IMPLANTABLE FLUID PUMP SYSTEM
SYSTÈME DE POMPE À FLUIDE IMPLANTABLE

(30) Priorität: 27.11.2015 DE 102015223541
(43) Veröffentlichungstag der Anmeldung: 03.10.2018
(73) Patentinhaber: Albert-Ludwigs-Universität Freiburg, 79098 Freiburg (DE)
(72) Erfinder: BOTHE, Wolfgang, 79098 Freiburg (DE); BENK, Christoph, 79106 Freiburg (DE); BEYERSDORF, Friedhelm, 79104 Freiburg (DE)
(74) Vertreter: Rösler, Uwe
(86) Internationale Anmeldenummer: PCT/EP2016/078630
(87) Internationale Veröffentlichungsnummer: WO 2017/089440

(56) Entgegenhaltungen:
- WO-A1-2012/165429
- WO-A1-2016/077444
- WO-A2-2007/067792
- WO-A2-2016/100600
- US-A1- 2013 133 944

## Beschreibung

### Technisches Gebiet

Die Erfindung bezieht sich auf ein implantierbares Fluidpumpensystem zur Strömungsunterstützung oder -initiierung innerhalb eines Fluid-durchströmten Hohlorgans, insbesondere des Herzens.

### Stand der Technik

Implantierbare Fluidpumpensysteme der vorstehend genannten Gattung dienen vornehmlich als Herzunterstützungssysteme, die im Englischen als VAD (Ventricular Assist Device) bezeichnet werden. Derartige implantierbare zur Herzunterstützung dienende Fluidpumpensysteme werden in Fällen einer schwerwiegenden Herzerkrankung, durch die die Blutversorgung des Körpers nicht mehr ausreichend gewährleistet ist, in die linke, rechte oder beide Herzkammern implantiert. In den meisten Fällen werden derartige, auch als Kunstherz bezeichnete Fluidpumpensysteme implantiert, um die Zeit bis zu einer Herztransplantation zu überbrücken. Im Falle einer linksventrikulären Herzinsuffizient wird das Fluidpumpensystem in die linke Herzkammer implantiert, wobei das Pumpensystem über einen entsprechenden intrakardial verlegten Tubus mit der Aorta verbunden ist, um Blut aus der linken Herzkammer in die Aorta pulsierend oder kontinuierlich, je nach eingesetztem Pumpmechanismus zu fördern.

Moderne miniaturisierte, intrakardiale Fluidpumpensysteme erlauben eine intrakardiale Implantation unter Einsatz minimalinvasiver Operationstechniken. Auf diese Weise können aufwendige chirurgischer Eingriffe, wie bspw. Anastomose an Ventrikelmyokard und Aorta sowie der Einsatz einer Herz-Lungen-Maschine vermieden werden.

Aus der Druckschrift US 2010/0249489 A1 geht ein, modernes intrakardiales Fluidpumpensystem hervor, das unter Verwendung der Seldingertechnik über die Herzspitze mit einer Verankerung im Bereich der Herzklappen ohne Verwendung einer Herz-Lungen-Maschine und ohne Anastome an der Aorta intrakardial implantierbar ist. Das bekannte intraventrikulare Blutpumpensystem verfügt über eine innerhalb eines Tubus eingesetzte motorisch angetriebene Fluidpumpe mit axialem Fluiddurchsatz. Der Tubus weist innerhalb des Ventrikellumens ein offenes Tubusende auf, über das Blut eingesaugt wird. Das dem offenen Tubusende axial gegenüberliegende Tubusende ist mit einem schlauchartigen Fortsatz fluiddicht verbunden, der durch den Bereich der Herzklappen in die Aorta offen mündet. Zur intrakardialen Fixierung des schlauchartigen Fortsatzes samt Tubus ist im Bereich des schlauchartigen Fortsatzes ein die Herzklappenfunktion ersetzendes Dilatationselement angebracht, das für einen festen Sitz der intrakardialen Fluidpumpenanordnung sorgt. Zur elektrischen Energieversorgung sowie auch Ansteuerung der elektromotorisch angetriebenen Fluidpumpe dient eine elektrische Zuleitung, die extrakardial durch eine implantationsbedingte apikal im Myokard eingebrachte Läsion verläuft, die mit Hilfe eines chirurgischen Fadens fluiddicht verschlossen ist.

Ein alternatives ventrikuläres Unterstützungssystem ist der Druckschrift US 8,852,072 B2 zu entnehmen, das über eine vollständig in den Ventrikel eingebrachte axiale Fluidpumpe umfasst, an der auswurfseitig ein tubenartiger Fortsatz angebracht ist, der durch den Bereich der Herzklappen hindurchragend in die Aorta mündet. Zu Zwecken der intrakardialen Fixierung sowie insbesondere einer beabstandeten intrakardialen Anordnung des Pumpeneinsaugtraktes vom Endokard, stützt sich das gesamte intrakardiale Fluidpumpensystem über ein stangenartig ausgebildetes Distanzelement an einer Abstützplatte ab, die chirurgisch am Myokard befestigt ist und zudem die implantationsbedingte apikale Läsion im Myokard fluiddicht abschließt. Zur Bewerkstelligung einer intrakardialen Beweglichkeit, um die Dynamik der Herzmuskelbewegung nicht oder möglichst geringfügig zu beeinträchtigen, ist das stangenförmige Distanzelement mit der myokardseitig fixierten Abstützplatte über ein Kugelgelenk verbunden.

Aus der Druckschrift US 2001/0041934 A1 ist ein künstliches Herzunterstützungssystem zu entnehmen, bei dem die Pumpe innerhalb des kardialen Lumens angeordnet ist. Das bekannte Pumpensystem ist dabei fest mit dem Gehäuse verankert, so dass ein selektiver Pumpentausch aus technischen Gründen nicht möglich.

Die Druckschrift US 02014/0364880 A1 offenbart eine konventionelle Blutpumpe, mit der Blut aus einer Kammer nach extrakardial und mithilfe einer Pumpe über eine lange Gefäßprothese in eine andere Kammer wieder eingeleitet wird. Die in der Druckschrift offenbarte Befestigungsanordnung für Pumpensysteme am Herzmuskel entspricht marktgängigen Konstruktionen.

Die Druckschrift WO 2014 / 145 667 A2 offenbart ein Blutpumpensystem in der abführenden Hauptschlagader.

Die Druckschrift US 2013 / 0 133 944 A1 beschreibt ein Pumpensystem mit extrakavitärer Anbringung von Motor und Pumpe. Bei extrakorporalen Herzunterstützungssystemen werden drei Röhren aus dem Körper geleitet, in Form eines Blutzufluss, Blutabfluss sowie eines Stromkabels. Es wird vorgeschlagen, alle Röhren aus ein- und demselben Zugang aus dem Körper auszuleiten, um die chirurgische Belastung für den Patienten zu minimieren.

Die Druckschrift WO 2007 / 103 464 A2 beschreibt ein Pumpensystem mit extrakavitärer Anbringung von Motor und Pumpe. Im Wesentlichen handelt es sich um eine Blutpumpe, die vornehmlich als Dialysepumpe eingesetzt wird.

Der Druckschrift WO2012/165429 A1 ist ein implantierbares Fluidpumpensystem zur Strömungsunterstützung innerhalb des Herzens zu entnehmen. Beschrieben ist eine doppellumige Kanüle, die die distalseitig durch die Herzwand ragt und extrakavitär mit einer Blutpumpe verbunden ist.

Die Druckschrift WO 2007/067792 A2 beschreibt ein Befestigungsmodul, das zur Anbringung an der Herzmuskelaußenwand geeignet ist und eine lösbar feste Verbindung zu einer die Herzfunktion unterstützenden Pumpe ermöglicht.

Gleichwohl die bekannten minimalinvasiv implantierbaren intraventrikularen Blutpumpensysteme zu Zwecken der Implantation in einer patientenschonenden Operationstechnik implantierbar sind, können nachträgliche Manipulationen an den vollständig intrakardial eingebrachten ventrikulären Unterstützungssystemen nur im Wege einer vollständigen Explantation vorgenommen werden. Neben den für den Patienten hierdurch grundsätzlich belastenden minimalinvasiven Eingriff erfahren vor allem die mit den intravaskulären Unterstützungssystem in Kontakt tretenden Herzklappen und/oder die an die Herzklappen angrenzenden Gefäßwandabschnitte eine erhebliche, zum Teil irreversible Irritation, die es grundsätzlich zu minimieren bzw. gänzlich zu vermeiden gilt.

### Darstellung der Erfindung

Der Erfindung liegt die Aufgabe zugrunde, ein implantierbares Fluidpumpensystem zur Strömungsunterstützung oder -initiierung innerhalb eines Fluid-durchströmten Hohlorgans, insbesondere des Herzens derart anzugeben, so dass zum einen, gleichsam den vorstehend beschriebenen bekannten intravaskulären Unterstützungssystemen, eine minimalinvasive und damit patientenschonende Implantationstechnik ohne die Notwendigkeit einer Herz-Lungen-Maschine anwendbar ist, zum anderen jedoch die Möglichkeit geschaffen werden soll, zumindest einzelne Komponenten, wie insbesondere die motorisch angetriebene Fluidpumpe in Fällen eines erforderlichen Austausches zu explantieren, ohne dabei intrakardial implantierte Komponenten des Fluidpumpensystems zu dislozieren und damit Herzklappen oder Herzklappenbereiche oder ähnliche intrakardiale Bereiche zu irritieren.

Die Lösung der der Erfindung zugrundeliegenden Aufgabe ist im Anspruch 1 angegeben. Den Erfindungsgedanken in vorteilhafter Weise weiterbildende Merkmale sind Gegenstand der Unteransprüche sowie der weiteren Beschreibung unter Bezugnahme auf das Ausführungsbeispiel zu entnehmen.

Das lösungsgemäße implantierbare Fluidpumpensystem, das zur Strömungsunterstützung oder -initiierung innerhalb eines beliebigen fluid-durchströmten intrakorporalen Hohlorgans eines Menschen oder eines Tieres einsetzbar ist, jedoch in besonders vorteilhafter Weise zur minimalinvasiven Implantation an einem menschlichen Herz geeignet ist, zeichnet sich grundsätzlich durch seinen modularen Aufbau aus, der es gestattet, den minimalinvasiven Implantationsvorgang in einer für den Patienten äußert schonenden Weise durchzuführen, bei der vereinzelte Komponenten nach und nach an bzw. in das Hohlorgan implantiert und in Art eines Baukastenprinzips intrakorporal montiert werden. Auf diese Weise werden sämtliche vom Implantationsvorgang betroffene intrakorporale Gewebebereiche maximal geschont, d. h. minimal irritiert. In gleicher Weise gestattet der modulare Aufbau einen Zugang zur elektromotorisch angetriebenen Pumpe, die im Falle eines erforderlichen Austausches bei einem bereits implantierten Fluidpumpensystems ohne die Notwendigkeit das jeweils betroffene Hohlorgan, insbesondere im Falle des Herzens, das Myokard betreffend, minimalinvasiv lokal zu öffnen. Hierzu stützt sich das lösungsgemäß ausgebildete implantierbare Fluidpumpensystem unmittelbar am Umfangsrand einer minimalinvasiv in die Hohlorganwand eingebrachte Öffnung ab, die zugleich durch die sich durch die Öffnung erstreckende Fluidpumpensystem fluiddicht abgedichtet wird. Somit verfügt das lösungsgemäß ausgebildete implantierbare Fluidpumpensystem über Komponententeile, die intravaskulär angeordnet sind sowie andere Komponententeile, die extravaskulär angeordnet und zugänglich sind.

Das im Weiteren offenbarte implantierbare Fluidpumpensystem zur Strömungsunterstützung oder -initiierung innerhalb eines fluiddurchströmten Hohlorgans eignet sich in besonderer Weise als Herzunterstützungssystem. Die weitere Beschreibung bezieht sich somit überwiegend auf den Fall der Implantation eines Fluidpumpensystems im bzw. am Herzen, gleichwohl eine Applikation des Systems auch an oder in anderen Blut- oder Lymph-durchströmten Hohlorganen möglich ist. Im Falle einer nicht am Herz vorzunehmenden Implantation sind die im folgenden verwendeten Begriffe "intrakardial" und "extrakardial" in Zusammenhang mit Herz durch "intravasal" und "extravasal" zu ersetzen.

Das lösungsgemäße implantierbare Fluidpumpensystem verfügt wenigstens über ein Intrakardialmodul (bzw. Intravasalmodul), ein Befestigungsmodul, eine Haltemodul sowie über ein Pumpenmodul.

Das Intrakardialmodul umfasst zwei separate Fluidkanäle, die jeweils eine intrakardiale sowie eine extrakardiale Fluidkanalöffnung besitzen. Der Begriff "intrakardial" soll hierbei jene Fluidkanalöffnung der jeweiligen Fluidkanäle charakterisieren, die im implantierten Zustand des Fluidpumpensystems innerhalb des Herzens, d. h. innerhalb einer Herzkammer angeordnet ist, wohingegen der Begriff "extrakardial" jene Fluidkanalöffnung charakterisiert, die bedingt durch die körperliche Ausbildung und Anordnung des Intrakardialmoduls im implantierten Zustand außerhalb des Herzens angeordnet ist oder zumindest eine vom Herz abgewandte Öffnungsrichtung aufweist.

Zu Zwecken der Abstützung und Fixierung des gesamten Fluidpumpensystems am Herz bzw. innerhalb des Herzens dient das Befestigungsmodul, das über eine Fügekontur verfügt, über die das Intrakardialmodul mit dem Befestigungsmodul fluiddicht fügbar ist. Darüber hinaus sieht das Befestigungsmodul eine Befestigungsstruktur zur intrakorporalen Befestigung am Herzen vor. In einer bevorzugten Ausführungsform ist das Befestigungsmodul grundsätzlich ringförmig bzw. tubusförmig ausgebildet, mit einer radial nach innen orientierten Mantelfläche, die der vorstehend bezeichneten Fügekontur zum fluiddichten Fügen an das Intrakardialmodul entspricht. Radial außen liegend sieht das Befestigungsmodul gleichfalls eine geeignet ausgebildete Fügekontur vor, an der im implantierten Zustand des lösungsgemäßen Fluidpumpensystems die Myokardwand einer apikal lokalen Läsion im Myokard peripher vollständig umfassend anliegt. Die Fügekontur ist vorzugsweise zumindest bereichsweise geradzylinderförmig ausgebildet. Alternativ sind auch von der Zylinderform abweichende Fügekonturen denkbar, bspw. konische, prismatische, d.h. n-eckige, o.ä.. Das Befestigungsmodul lässt sich mit einer geeignet gewählten chirurgischen Befestigungstechnik, bspw. mit Hilfe eines chirurgischen Fadens oder chirurgischer Klammern am Myokard befestigen. Weitere Einzelheiten hierzu sind in Verbindung mit der Beschreibung eines konkreten Ausführungsbeispiels zu entnehmen.

Eine bevorzugte Modifikation des Befestigungsmoduls sieht eine Unterdruck- bzw. Sog-unterstützte temporäre Fixierung des Befestigungsmoduls an der Myokardaußenwand vor, die es dem Operateur ermöglicht eine Positionierung und erforderlichenfalls eine für die Myokardwand schadlose Nachjustierung exakt vorzunehmen. Nach korrekter Anbringung des Befestigungsmoduls am Myokard erfolgt mittels Klebetechnik eine dauerhafte Fixierung des Moduls. Hierzu sieht das Befestigungsmodul mehrere der Myokardwand zugewandte nutförmige Ausnehmungen vor. Zum Zwecke einer temporären Anhaftung des Befestigungsmoduls an der Myokardwand wird längs wenigstens einer ausgewählten Ausnehmung ein Unterdruck appliziert. Im Zustand der Sog-unterstützten Anhaftung wird sodann längs wenigstens einer weiteren der Myokardwand zugewandten nutförmigen Ausnehmung biokompatibler Klebstoff injiziert, der das Befestigungsmodul dauerhaft an das Myokard zu fixieren vermag. Zudem stellt die Klebeverbindung eine luftdichte Fügeverbindung zwischen dem Befestigungsmodul und der Myokardwand her, durch die Luftembolien ausgeschlossen werden können.

Im gefügten Zustand umschließt das Befestigungsmodul das Intrakardialmodul längs der jeweils gegenkonturiert ausgebildeten Fügekonturen. Die Fügekontur des Intrakardialmoduls befindet sich in einem medialen Abschnittes des Intrakardialmoduls, das in einer axialen Abfolge einen extrakardialen, einen medialen sowie einen intrakardialen Abschnitt aufweist. Die einzelnen axialen Abschnitte des Intrakardialmoduls sind lediglich durch ihre Lage relativ zum Herzen im implantierten Zustand charakterisiert und ansonsten vorzugsweise einstückig miteinander verbunden und vorzugsweise aus einem einheitlichen Material gefertigt.

In einem bevorzugten Ausführungsbeispiel weist der intrakardiale Abschnitt des Intrakardialmoduls, der im implantierten Zustand innerhalb einer Herzkammer zu liegen kommt, sowohl einen tubenartigen Fortsatz mit einem offenen distalen Tubenende, der einen der beiden Fluidkanäle umfasst, sowie lateral zum tubenartigen Fortsatz beabstandet, die intrakardiale Fluidkanalöffnung des anderen Fluidkanals auf.

In einem anderen Ausführungsbeispiel ist der andere Fluidkanal zumindest teilweise als Ringkanal ausgebildet und koaxial um den tubenartigen Fortsatz, der wie im vorstehenden Ausführungsbeispiel den einen der beiden Fluidkanäle umschließt, angeordnet. In beiden Fällen münden die beiden Fluidkanäle getrennt voneinander jeweils den intrakardialen Fluidkanalöffnungen gegenüberliegend, im Bereich des extrakardialen Abschnittes des Intrakardialmoduls, der im implantierten Zustand außerhalb des Herzens angeordnet ist und somit extrakardial frei zugänglich ist.

Das Pumpenmodul, das fluiddicht an die extrakardialen Fluidkanalöffnungen zur Herstellung einer fluiddichten Verbindung beider Fluidkanäle miteinander anschließbar ist, lässt sich auf dieser Weise am extrakardialen Abschnitt des Intrakardialmoduls mittel- oder unmittelbar lösbarfest fixieren.

Das Pumpenmodul weist in einer bevorzugten Ausführungsform ein U-förmiges Rohrleitungssystem auf, längs dem eine motorisch angetriebene Fluidförderpumpe eingebracht ist. In vorteilhafter Weise weist das U-förmige Rohrleitungssystem zwei offen mündende Rohrleitungsstutzen auf, die im Wege einer fluiddichten Steckverbindung mit den extrakardialen Fluidkanalöffnungen der vom Intrakardialmodul umfassten Fluidkanäle fluiddicht und lösbarfest verbindbar sind.

Zur elektrischen Energieversorgung sowie auch zu Zwecken der Pumpenansteuerung ist das Pumpenmodul vorzugsweise mit einer Kabelzu- bzw. - abführung verbunden, die aufgrund der extrakardialen Anbringung des Pumpenmoduls ebenfalls außerhalb des Herzens verläuft und somit das Myokard nicht penetriert, wie dies jedoch bei an sich bekannten gattungsgemäßen Vorrichtungen in nachteilhafter Weise der Fall ist.

Sollte es nach einem langjährigen Einsatz des implantierten Fluidpumpensystems zu einem Pumpendefekt oder einer ähnlichen technischen Störung kommen, so ist es ohne dabei das Herz selbst nennenswert zu irritieren mit Hilfe eines minimalinvasiven Eingriffes möglich, das Pumpenmodul zu warten oder zu ersetzen.

Zur Erläuterung zweier bevorzugter Ausführungsbeispiele für ein sämtliche Komponenten umfassendes Fluidpumpensystem wird im Weiteren auf die Figurenbeschreibungen hingewiesen.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: Schematisierte Gesamtdarstellung eines ersten implantierbaren Fluidpumpensystems,
- Fig. 2a,b: Perspektivische Darstellungen eines ersten intrakardialen Moduls,
- Fig. 3: Darstellung eines ersten Befestigungsmoduls,
- Fig. 4: Darstellung eines ersten Haltemoduls,
- Fig. 5a,b: Darstellung eines Pumpenmoduls,
- Fig. 6: Schematisierte Darstellung eines zweiten implantierbaren Fluidpumpensystems ohne Darstellung des Pumpenmoduls,
- Fig. 7: Perspektivische Darstellungen eines zweiten intrakardialen Moduls,
- Fig. 8: Darstellung eines zweiten Befestigungsmoduls,
- Fig. 9a, b, c: Darstellung einer Modifikation das Befestigungsmodul betreffend sowie
- Fig. 10: Darstellung eines chirurgischen Werkzeuges zur Einbringung einer lokalen Läsion innerhalb des Myokards zu Zwecken der Implantation des Fluidpumpensystems

### Wege zur Ausführung der Erfindung, gewerbliche Verwendbarkeit

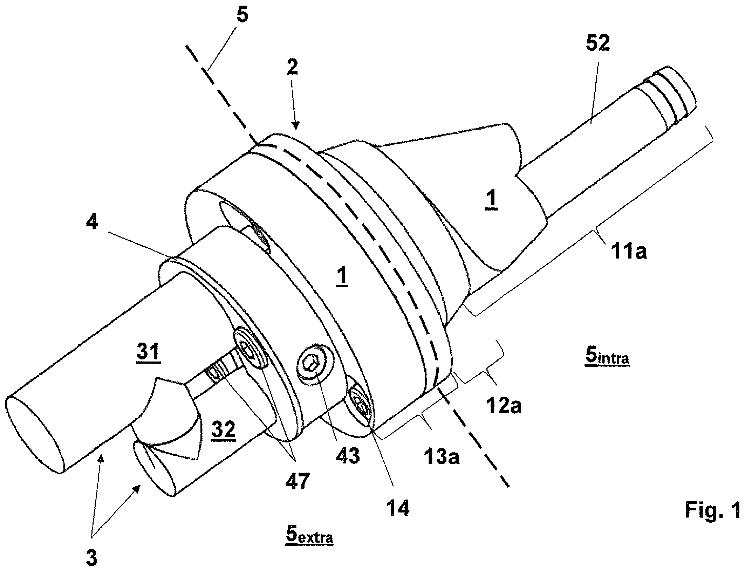

Figur 1 zeigt eine schematische perspektivische Darstellung eines Ausführungsbeispiels eines implantierbaren Fluidpumpensystems, das zu Zwecken der Herzfunktionsunterstützung auf minimalinvasive Weise apikal in das Myokard eines Herzen implantierbar ist. Das Fluidpumpensystem ist modular aufgebaut und setzt sich aus den folgenden einzelnen Modulen zusammen: Intrakardialmodul 1, Befestigungsmodul 2, Pumpenmodul 3 sowie Haltemodul 4.

Sämtliche Module lassen sich sowohl zu Zwecken der Implantation sowie aber auch der Explantation vereinzelt, d.h. als Einzelkomponenten handhaben. Das Zusammenfügen der einzelnen Komponenten erfolgt jeweils durch bloßes Zusammenfügen bzw. Ineinanderstecken, wobei zu Zwecken einer gegenseitigen Fixierung jeweils zweier unmittelbar in Kontakt tretender Komponenten für einen Operateur leicht zugänglicher Schraubverbindungen zu Einsatz kommen.

Das in Figur 1 illustrierte, die Herzfunktion unterstützende Fluidpumpensystem zeichnet sich neben seinem modularen Aufbau insbesondere dadurch aus, dass über das Befestigungsmodul 2 sämtliche Komponenten des Fluidpumpensystems, die ansonsten nicht notwendigerweise mit dem Herzen in Kontakt treten, relativ zum Herz gelagert bzw. fixiert werden. Zudem sorgt das Befestigungsmodul 2 für eine fluiddichte Abdichtung der implantationsbedingten Öffnung innerhalb des Myokards, das in der Bilddarstellung gemäß Figur 1 stark schematisiert durch die strichpunktierte Linie 5 dargestellt ist, die den intrakardialen Bereich 5ᵢₙₜᵣₐ vom extrakardialen Bereich 5ₑₓₜᵣₐ trennt. Somit weist das Fluidpumpensystem Komponententeile auf, die im implantierten Zustand eine intrakardiale Lage besitzen, dies betrifft insbesondere den intrakardialen Abschnitt 11a des Intrakardialmoduls 1, sowie Komponententeile, die außerhalb des Herzens angeordnet sind, dies betrifft insbesondere den extrakardialen Abschnitt 13a des Intrakardialmoduls 1, das Pumpenmodul 3 sowie ein das Pumpenmodul 3 mit dem extrakardialen Abschnitt 13a des Intrakardialmoduls 1 fluiddicht fixierenden Haltemoduls 4.

Zur Erläuterung der einzelnen Module sei im Weiteren auf die Einzelbilddarstellungen der weiteren Figuren Bezug genommen.

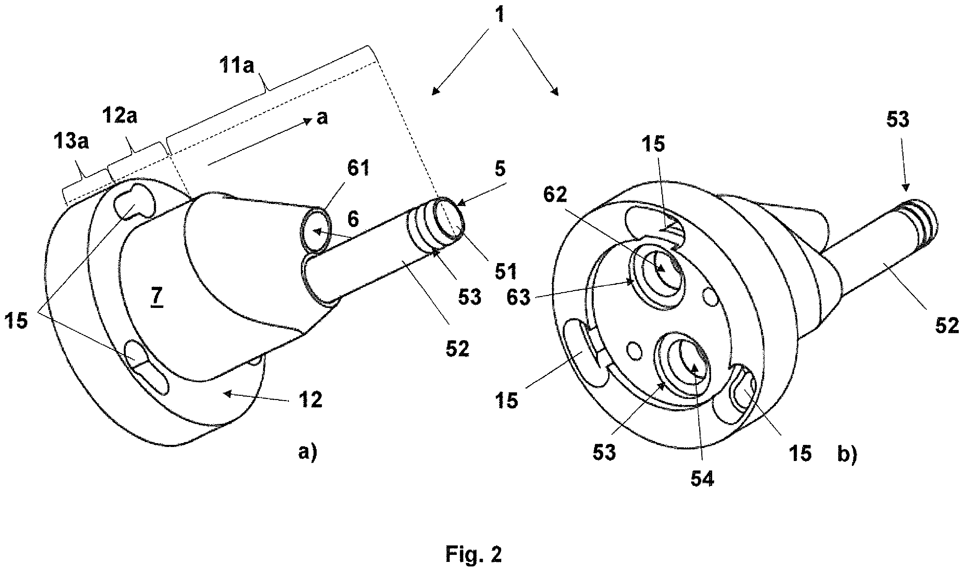

Die Figuren 2a, b zeigen in unterschiedlichen perspektivischen Ansichten das Intrakardialmodul 1 in Alleindarstellung, das, wie bereits vorstehend erwähnt, in axialer Abfolge längs einer Raumrichtung a einen extrakardialen Abschnitt 13a, einen sich an diesen unmittelbar anschließenden medialen Abschnitt 12a sowie einen intrakardialen Abschnitt 11a aufweist. Das Intrakardialmodul 1 ist vorzugsweise, gleichsam wie das Befestigungsmodul 2 sowie auch das Haltemodul ,4 einstückig aus einem bioverträglichen Material gefertigt. Im Wesentlichen umfasst das Intrakardialmodul 1 zwei Fluidkanäle 5, 6, die jeweils offene intrakardiale Fluidkanalöffnungen 51, 61 aufweisen. Der erste Fluidkanal 5 weist einen tubenartigen Fortsatz 52 auf, dessen Länge in Abhängigkeit anatomischer Verhältnisse gewählt ist. In einem bevorzugten Ausführungsbeispiel weist der tubenartige Fortsatz 52 eine Länge auf, die derart gewählt ist, so dass im implantierten Zustand des Fluidpumpensystems die Fluidkanalöffnung 51 im Bereich der Aorta mündet. Alternativ ist es möglich, die Länge des tubenartigen Fortsatzes 52 entsprechend kurz zu wählen. Über eine fluiddichte Verbindungsstruktur 53, die im Bereich der Fluidkanalöffnung 51 vorgesehen ist, kann ein künstlicher Anschlußtubus, der nicht in Figur 2 dargestellt ist, fluiddicht und lösbar fest an dem tubenartigen Fortsatz 52 angebracht werden, um so eine direkte Versorgung der Aorta mit Blut zu gewährleisten. Die Ausbildung der Verbindungsstruktur 53 ist insbesondere in jenen Fällen vorteilhaft, in denen der künstliche Anschlußtubus vorzugsweise im Bereich der Herzklappen, bspw. mit Hilfe eines Dilatationselements fixiert ist.

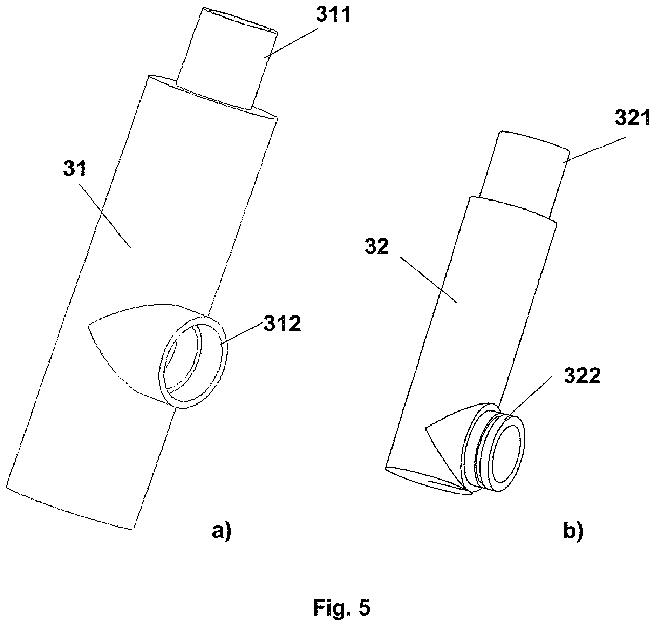

Lateral neben dem ersten Fluidkanal 5 sieht das Intrakardialmodul 1 die Fluidkanalöffnung 61 des zweiten Fluidkanals 6 vor, die offen innerhalb der Herzkammer mündet. Beide Fluidkanäle 5, 6 durchragen separat das Intrakardialmodul 1 und münden offen im Bereich des extrakardialen Abschnittes 13a an den in Figur 2b ersichtlichen, extrakardialen Fluidkanalöffnungen 54, 62. Die extrakardialen Fluidkanalöffnungen 54, 62 verfügen jeweils über eine stufenartig koaxial ausgebildete Einsteckkontur 53, 63, die in Art einer Steckdosenfunktion ein fluiddichtes Einstecken jeweils gegenkonturiert ausgebildeter Rohrleitungsstutzen ermöglicht, auf die in Verbindung mit der Beschreibung des in Figur 5a, b illustrierten Pumpenmoduls Bezug genommen wird.

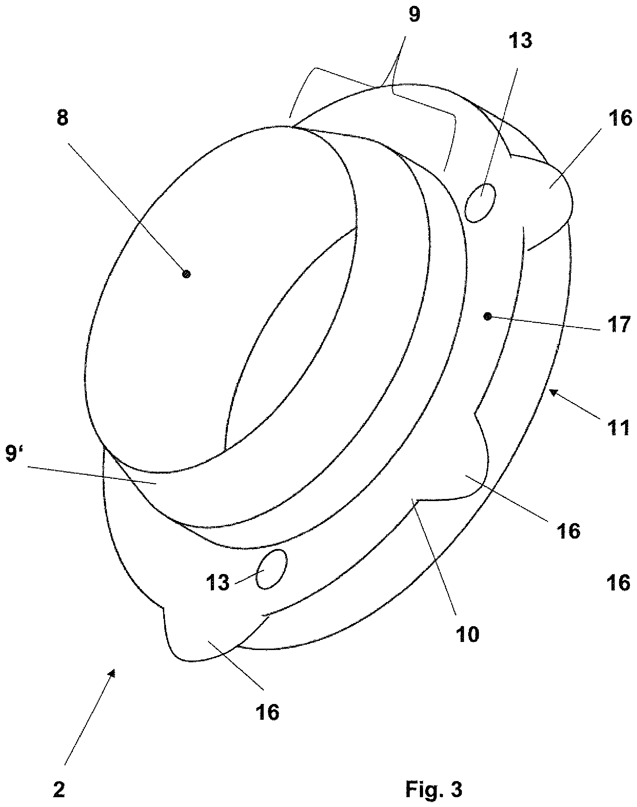

Das Intrakardialmodul 1 weist im Bereich des so genannten medialen Abschnittes 12a eine zylinderförmig ausgebildete Außenkontur auf, die als Fügekontur 7 des Intrakardialmoduls 1 dient. Die zylinderförmig ausgebildete Fügekontur 7 ist passgenau und gegenkonturiert zur Fügekontur 8 des in Figur 3 dargestellten Befestigungsmoduls 2 ausgebildet. Das Befestigungsmodul 2 verfügt vorzugsweise über eine ringförmig, propfenartige Raumform mit einem tubusförmigen Abschnitt 9, der radial innenseitig wenigstens teilweise die Fügekontur 8 umfasst und an dem sich einseitig ein dem tubusförmigen Abschnitt 9 radial überragender Kragen 10 anschließt. Vorzugsweise erstreckt sich die geradzylinderförmig ausgebildete Fügekontur 8 über die gesamte axiale Länge des Befestigungsmoduls 2 und liegt somit über ihre gesamte axiale Länge im gefügten Zustand an der Fügekontur 7 des Intrakardialmoduls 1 fluiddicht an.

Zu Zwecken einer schonenden Ein- bzw. Durchführung des tubusförmigen Abschnittes 9 durch eine lochartig ausgebildete Läsion innerhalb des Myokards ist der tubusförmige Abschnitt 9 in einen den Kragen 10 abgewandten Endbereich 9' konisch verjüngend ausgeführt.

Ferner verfügt der Kragen 10 eine dem tubusförmigen Abschnitt 9 abgewandte Stirnfläche 11 sowie eine dem tubusförmigen Abschnitt 9 zugewandte Stirnfläche 17.

Die Stirnfläche 11 ist ringscheibenartig ausgebildet und in Form und Größe einer am extrakardialen Abschnitt 13a des Intrakardialmoduls 1 vorgesehenen Abstützfläche 12, siehe Figur 2a, angepasst, an der die Stirnfläche 11 des Befestigungsmoduls 2 im gefügten Zustand zwischen Intrakardialmodul 1 und Befestigungsmodul 2 bündig anliegt, siehe hierzu insbesondere Gesamtdarstellung gemäß Figur 1.

Zu Zwecken einer gegenseitigen lösbar festen Fixierung zwischen Befestigungsmodul 2 und Intrakardialmodul 1 sieht das Befestigungsmodul 2 im Bereich des radial überragenden Kragens 10 Ausnehmungen 13 mit einem Innengewinde vor, die vorzugsweise als Sackloch- oder vollständig den Kragen 10 durchragende Bohrungen ausgebildet sind. Mit Hilfe passender Schrauben 14, siehe Figur 1, die über den extrakardialen Abschnitt 13a des Intrakardialmoduls 1 durch entsprechend vorgesehene Öffnungen 15 einsetzbar sind, gelangen diese in Eingriff mit dem jeweiligen Innengewinde der Ausnehmungen 13.

Zu Zwecken der Befestigung des Befestigungsmoduls 2 am Myokard weist das Befestigungsmodul 2 Befestigungsstrukturen 16 auf, die vorzugsweise an der dem tubusförmigen Abschnitt 9 zugewandten ringförmigen Stirnfläche 17 angebracht sind. Die Befestigungsstrukturen 16 sind vorzugsweise als den Kragen 10 radial, zumindest teilweise überragende Flächenelemente ausgebildet, die mit Hilfe einer chirurgischen Nadel durchstochen werden können, um auf diese Weise mittels eines chirurgischen Fadens am umliegenden Myokard fixiert zu werden. Die Befestigungsstrukturen sind beispielsweise aus einem künstlichen Gewebematerial oder einem Vlies ausgebildet, das unmittelbar mit dem Epikard in Anlage gebracht werden kann.

Das in Figur 1 dargestellte Pumpenmodul 3 verfügt über ein U-förmiges Rohrleitungssystem, längs dem eine motorisch, nicht weiter dargestellte angetriebene Fluidförderpumpe eingebracht ist. Das U-förmige Rohrleitungssystem des Pumpenmoduls verfügt über zwei offen endende Rohrleitungsstutzen, die derart angeordnet und ausgebildet sind, so dass sie durch bloßes axiales Fügen in die stufenförmig ausgebildete Steckkonturen 53, 63 der extrakardialen Fluidkanalöffnungen 54, 62 fluiddicht fügbar sind.

In vorteilhafter Weise ist das U-förmig ausgebildete Rohrleitungssystem des Pumpenmoduls 3 zweiteilig ausgebildet und weist zwei trennbare Rohrleitungsabschnitte 31, 32 auf, die sowohl in der Gesamtdarstellung gemäß Figur 1 als auch in Figur 5a, b illustriert sind. Im Rohrabschnitt 31, der stirnseitig oben offen mündet und eine stufenförmig ausgebildete Steckerkontur 331 aufweist, die zur fluiddichten Fügung in die gegenkonturiert stufenförmig ausgebildeten Einsteckkontur 63 fügbar ist, ist eine nicht weiter dargestellte motorisch angetriebene Fluidpumpe integriert, die Blut über das oben offene Ende des Rohrabschnittes 31 ansaugt. Der Rohrabschnitt 31 verfügt darüber hinaus über einen seitlichen zur Rohrlängsachse des Rohrabschnittes 31 orientierten offen ausgebildeten Rohrstutzen 312, der eine fluiddichte Verbindungsstruktur zu einem entsprechend am zweiten Rohrabschnitt 32 vorgesehenen Rohrstutzen 322 vorsieht. Vorzugsweise sind beide Rohrstutzen 31, 32 über ihre Rohrstutzen 312, 322 in Art einer Steckverbindung unter Verwendung einer O-Ring-Dichtung fluiddicht miteinander verbindbar. Auch der zweite Rohrabschnitt 32 weist an seinem oberen Ende eine stufenförmig ausgebildete Steckkontur 321 auf, die entsprechend gegenkonturiert zur stufenförmigen Einsteckkontur 53 der extrakardialen Fluidkanalöffnung 54 des ersten Fluidkanals 5 ausgebildet ist.

Das vorstehend beschriebene Pumpenmodul 3 vermag somit Blut über den zweiten Fluidkanal 6 aus der Herzkammer durch das U-förmige Rohrleitungssystem des Pumpenmoduls 3 in Richtung des ersten Fluidkanals 5 zu fördern, aus dem das Blut über den tubenförmigen Fortsatz 52, dessen distales Tubenende 51 im Bereich der Aorta liegt oder mit einem Verlängerungstubus verbunden ist, zu fördern.

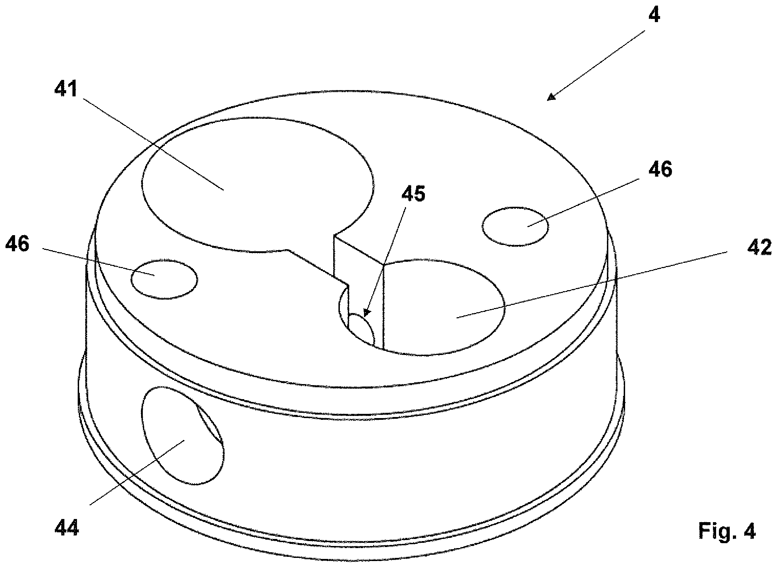

Aus Gründen einer sicheren und mechanisch belastbaren Fügung des Pumpenmoduls 3 an die entsprechenden extrakardialen Fluidkanalöffnungen 54, 62 ist ein Haltemodul 4 vorgesehen, das in Figur 4 illustriert ist. Das Haltemodul 4 dient zum einen einer gegenseitigen Befestigung der beiden Rohrabschnitte 31, 32. Hierzu sieht das im Wesentlichen scheibenförmig ausgebildete Haltemodul 4 zwei Durchtrittsöffnungen 41, 42 vor, die das Haltemodul 4 vollständig durchragen und deren Innendurchmesser jeweils an den Außendurchmesser der Rohrabschnitte 31, bzw. 32 angepasst sind. Aus Figur 4 geht hervor, dass der die Fluidpumpe umfassende Rohrabschnitt 31, der durch die Durchtrittsöffnung 41 hindurchgeschoben wird, einen größeren Durchmesser besitzt, als der Rohrabschnitt 32. Beide Rohrabschnitte 31, 32 werden zu Zwecken der Montage lediglich durch die entsprechenden Durchtrittsöffnungen 41, 42 hindurchgeschoben. Mit Hilfe einer Fixierschraube 43, siehe hierzu Figur 1, die durch eine seitlich im Haltemodul 4 eingebrachte Schraubenöffnung 44 einsetzbar und in Eingriff mit einem innerhalb des Haltemoduls 4 vorgesehenen Innengewindes 45 bringbar ist, werden beide Rohrabschnitte 31, 32 im Wege einer Klemmkraft-beaufschlagten Formschlussverbindung gegeneinander fest gefügt. Das Haltemodul 4 weist darüber hinaus weitere Befestigungsöffnungen 46 auf, in die Befestigungsschrauben 47, siehe Figur 1, zur lösbar festen Fixierung des Haltemoduls 4 relativ zum Intrakardialmodul 1 einsetzbar sind.

In einer bevorzugten Ausführungsform ist der Rohrabschnitt 31 gesamtheitlich als eine bereits für den medizinischen Einsatz zertifizierte und zugelassene motorisch angetriebene Diagonalpumpe ausgebildet.

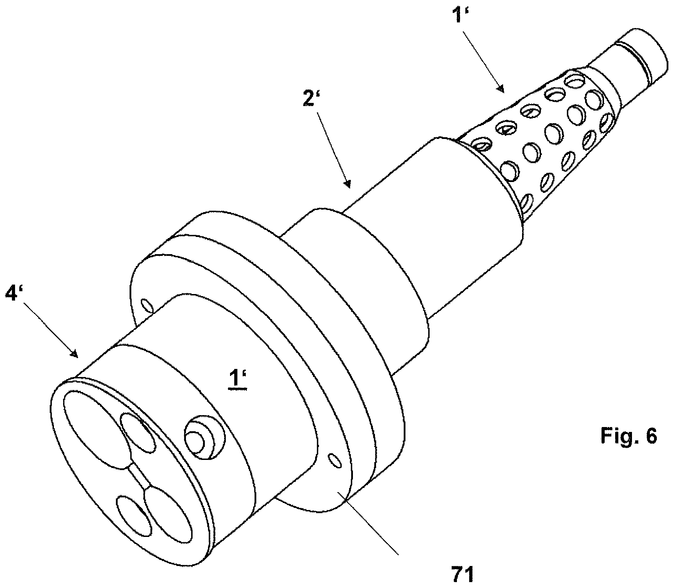

Figur 6 zeigt eine schematische perspektivische Darstellung eines zweiten Ausführungsbeispiels für ein implantierbares Fluidpumpensystems, das über die gleiche modulare Bauweise verfügt, wie das in Figur 1 illustrierte Ausführungsbeispiel. Es weist ein Intrakardialmodul 1', ein Befestigungsmodul 2', ein Haltemodul 4' sowie ein Pumpenmodul auf. Letzteres ist zu Zwecken einer besseren Übersichtlichkeit nicht dargestellt. Aufgrund der extrakardialseitig identischen Ausgestaltung des Haltemoduls 4' im Vergleich zu dem Haltemodul 4, das in Figur 4 näher erläutert ist, lässt sich das Pumpenmodul 3 in unveränderter Bauweise auch an das zweite Ausführungsbeispiel adaptieren, weshalb auf eine wiederholte Darstellung und Erläuterung des Pumpenmoduls 3 verzichtet wird.

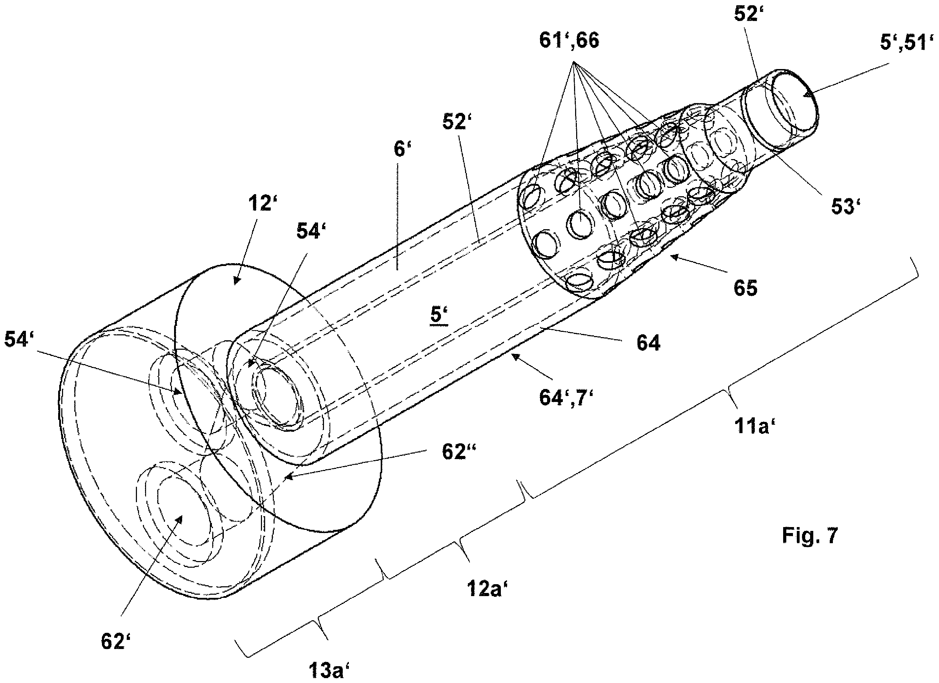

Im Unterschied zu dem in Figur 1 dargestellten ersten Fluidpumpensystem weist das zweite Ausführungsbeispiel eine modifizierte Bauform betreffend das Intrakardialmodul 1' auf, das in Alleinstellung in Figur 7 gezeigt ist.

Das Intrakardialmodul 1' zeichnet sich durch eine im Wesentlichen rotationssymmetrische äußere Raumform aus, die eine erleichterte Implantation ermöglicht, zumal eine möglicherweise zu beachtende intrakardiale Lageausrichtung des Intrakardialmoduls 1 im Falle des ersten Ausführungsbeispiels aufgrund der lateralen Anbringung des zweiten Fluidkanals 6 relativ zum ersten Fluidkanal 5, nun aufgrund einer koaxialen Anbringung des ersten Fluidkanals 5' und des zweiten Fluidkanals 6' entfällt.

Gleichsam dem Intrakardialmodul 1 in Figur 2 weist auch das Intrakardialmodul 1' gemäß Fig. 7 einen intrakardialen Abschnitt 11a', medialen Abschnitt 12a' und extrakardialen Abschnitt 13a' auf.

Das Intrakardialmodul 1' umfasst zwei Fluidkanäle 5', 6', die jeweils offene intrakardiale Fluidkanalöffnungen 51', 61' aufweisen. Der erste Fluidkanal 5' weist einen tubenartigen Fortsatz 52' auf, dessen Länge in Abhängigkeit anatomischer Verhältnisse gewählt ist. In einem bevorzugten Ausführungsbeispiel weist der tubenartige Fortsatz 52' eine Länge auf, die derart gewählt ist, so dass im implantierten Zustand des Fluidpumpensystems die Fluidkanalöffnung 51' im Bereich der Aorta mündet. Alternativ ist es möglich, die Länge des tubenartigen Fortsatzes 52' entsprechend kurz zu wählen. Über eine fluiddichte Verbindungsstruktur 53', die im Bereich der Fluidkanalöffnung 51' vorgesehen ist, kann ein künstlicher Anschlußtubus, der nicht in Figur 7 dargestellt ist, fluiddicht und lösbar fest an dem tubenartigen Fortsatz 52' angebracht werden, um so eine direkte Versorgung der Aorta mit Blut zu gewährleisten. Die Ausbildung der Verbindungsstruktur 53' ist insbesondere in jenen Fällen vorteilhaft, in denen der künstliche Anschlußtubus vorzugsweise im Bereich der Herzklappen, bspw. mit Hilfe eines Dilatationselements fixiert ist.

Radial um den tubenartigen Fortsatz 52' ist ein Aussenrohr 64 angeordnet, dessen axiale Erstreckung kleiner bemessen ist als der tubenartige Fortsatz 52' und das mit dem tubenartigen Fortsatz 52' einen ringkanalförmigen Zwischenraum einschließt, der dem zweiten Fluidkanal 6' zugeordnet ist. Das Aussenrohr 64 weist eine geradzylinderförmige Aussenmantelfläche 64' auf, die als Fügekontur 7' des Intrakardialmoduls 1' dient.

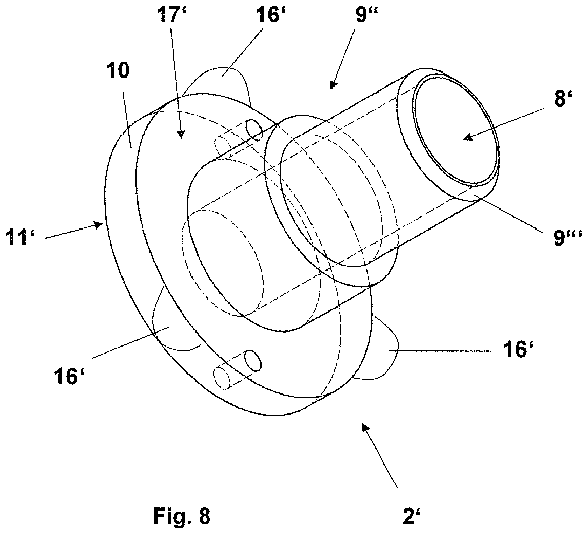

Die zylinderförmig ausgebildete Fügekontur 7' ist passgenau und gegenkonturiert zur Fügekontur 8' des in Figur 8 dargestellten Befestigungsmoduls 2' ausgebildet, auf das weiter unten eingegangen wird.

Das Aussenrohr 64 schließt einseitig an einer sich relativ zum Aussenrohr radial erweiternden Abstützfläche 12' ab, die einer Begrenzungsfläche des extrakardialen Abschnittes 13a' des Intrakardialmoduls 1 entspricht. Innerhalb des extrakardialen Abschnittes 13a' münden der ersten und zweite Fluidkanal 5', 6' über ihre jeweilige extrakardiale Fluidkanalöffnung 54', 62'. Hierzu ist der mittige Fluidkanal 5' über eine seitliche Abführung 54" mit der extrakardialen Fluidkanalöffnung 54' verbunden. Eine zweite seitliche Abführung 62" verbindet den ringförmigen Fluidkanal 6' mit der extrakardialen Fluidkanalöffnung 62' ohne dabei die seitliche Abführung 54" zu durchdringen.

An das Aussenrohr 64 schließt intrakardialseitig eine konische Übergangshülse 65 bündig an, deren Aussendurchmesser mit zunehmenden axialen Abstand zum Aussenrohr 64 auf die Dimension des Aussendurchmessers des tubenartigen Fortsatzes 52' abnimmt. Gleichfalls dem Aussenrohr 64 schließt auch die Übergangshülse 65 einen Zwischenraum mit dem tubenartigen Fortsatz 52' ein, der Teil des zweiten Fluidkanals 6' ist. Die Hülsenwand der Übergangshülse 65 verfügt über eine Vielzahl von Durchgangsöffnungen 66, über die ein Zugang zum zweiten Fluidkanal 6' geschaffen ist. Die Gesamtheit aller Durchgangsöffnungen 66 stellt die intrakardiale Fluidkanalöffnung 61' zum zweiten Fluidkanal 6' dar.

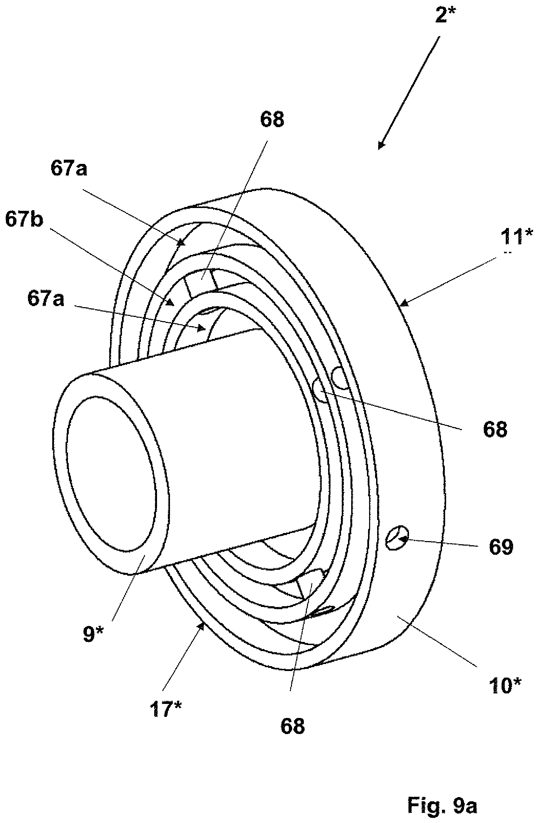

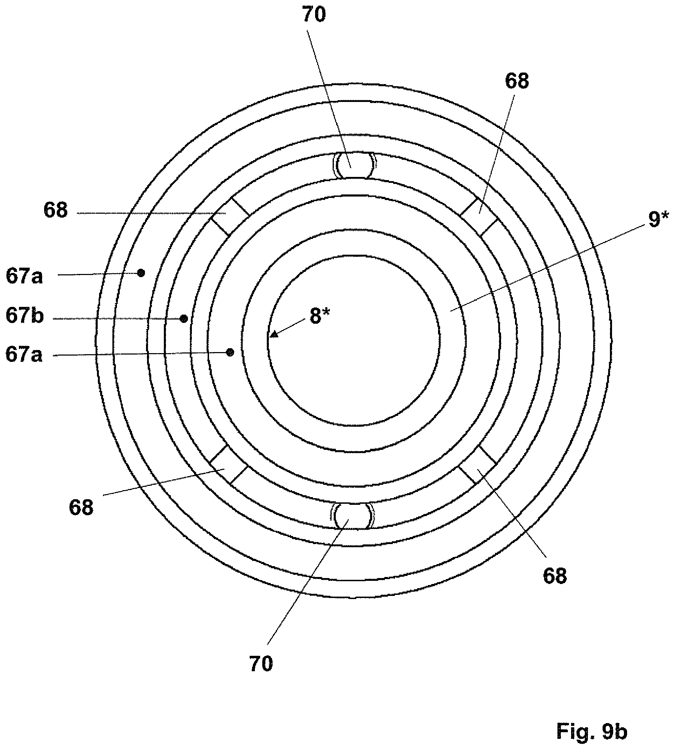

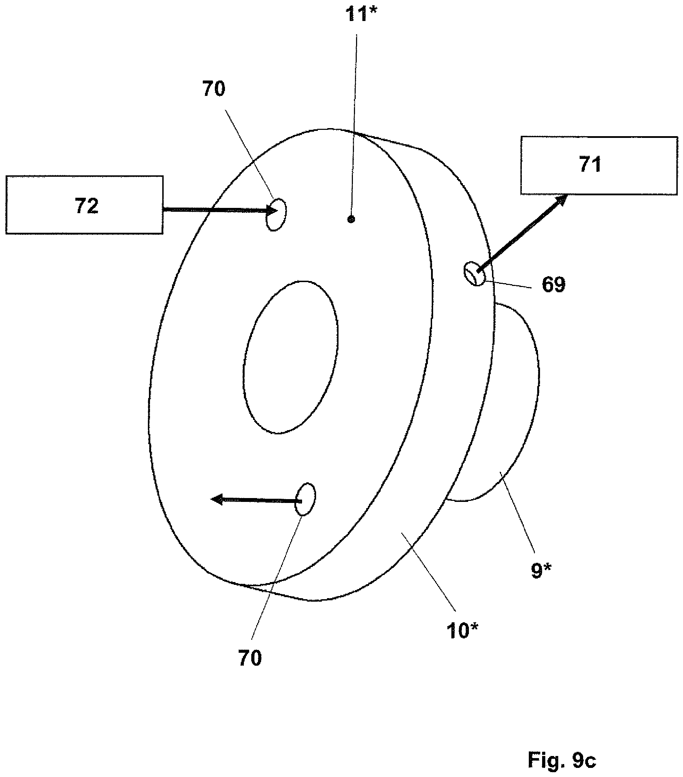

Für die Befestigung des Intrakardialmoduls 1' am Herz dient das Befestigungsmodul 2', das in Figur 9 dargestellt ist und vorzugsweise über eine ringförmig, propfenartige Raumform mit einem tubusförmigen Abschnitt 9" verfügt, der radial innenseitig die Fügekontur 8' umfasst und an dem sich einseitig ein dem tubusförmigen Abschnitt 9" radial überragender Kragen 10' anschließt. Vorzugsweise erstreckt sich die geradzylinderförmig ausgebildete Fügekontur 8' über die gesamte axiale Länge des Befestigungsmoduls 2' und liegt somit über ihre gesamte axiale Länge im gefügten Zustand an der Fügekontur 7' des Intrakardialmoduls 1' fluiddicht an.

Zu Zwecken einer schonenden Ein- bzw. Durchführung des tubusförmigen Abschnittes 9" durch eine lochartig ausgebildete Läsion innerhalb des Myokards ist der tubusförmige Abschnitt 9"' in einen den Kragen 10' abgewandten Endbereich 9" konisch verjüngend ausgeführt.

Ferner verfügt der Kragen 10' eine dem tubusförmigen Abschnitt 9" abgewandte Stirnfläche 11' sowie eine dem tubusförmigen Abschnitt 9" zugewandte Stirnfläche 17'.

Die Stirnfläche 11' ist ringscheibenartig ausgebildet und liegt im gefügten Zustand mit dem Intrakardialmodul 1' mittel- oder unmittelbar an der am extrakardialen Abschnitt 13a' des Intrakardialmoduls 1' vorgesehenen Abstützfläche 12' an. In der Gesamtdarstellung gemäß Figur 7 ist zusätzlich ein Zwischenring 71 zwischen der Abstützfläche 12' und dem Befestigungsmodul 2' eingebracht.

Sowohl zur Zwecken einer lösbar festen Montage des Intrakardialmoduls 1' mit dem Befestigungsmodul 2', als auch der Anbringung des Befestigungsmoduls 2' am Myokard sind die gleichen Vorkehrungen getroffen, wie im Ausführungsbeispiel gemäß Figur 1. So sind peripher um die Stützfläche 17' des Befestigungsmoduls 2' Befestigungsstrukturen 16 angebracht, die in Form von den Kragen 10' radial, zumindest teilweise überragende Flächenelemente ausgebildet sind, die mit Hilfe einer chirurgischen Nadel durchstochen werden können, um auf diese Weise mittels eines chirurgischen Fadens am umliegenden Myokard fixiert zu werden. Die Befestigungsstrukturen 16' sind beispielsweise aus einem künstlichen Gewebematerial oder einem Vlies ausgebildet, das unmittelbar mit dem Epikard in Anlage gebracht werden kann.

Ferner fügt sich das Haltemodul 4' in gleicher Weise extrakardialseitig an das Intrakardialmodul 1' an, wie im Falle des vorstehenden Ausführungsbeispiels. Das Haltemodul 4' erlaubt aufgrund seiner identischen Bauform die Anbringung des Pumpenmoduls 3, das in Figur 5 bereits erläutert worden ist.

In den Figuren 9a, b und c ist ein Befestigungsmodul 9* illustriert das einen modifizierten Kragen 10* besitzt, der über einen alternativen oder über einen zusätzlichen Fixierungsmechanismus zu den bereits beschriebenen Befestigungsstrukturen 16 verfügt. Zum Zwecke einer übersichtlicheren Darstellung und Erläuterung der Kragenmodifikation zeigt Figur 9a eine perspektivische Darstellung mit Blick auf die Stirnfläche 17* des Kragens 10*, wobei der tubusförmige Abschnitt 9* gleich dem Abschnitt 9 des Ausführungsbeispiels in Figur 3 bzw. 9" des Ausführungsbeispiels in Figur 8 ausgebildet sein kann. Figur 9b zeigt eine axiale Ansicht auf den Kragen 10*. Figur 9c zeigt eine rückwärtige Ansicht des Kragens 10* auf die Stirnseite 11*. Die weitere Beschreibung nimmt Bezug auf alle drei Bilddarstellungen gemäß der Figuren 9 bis c.

Der modifizierte Kragen 10*, weist nutförmige Ausnehmungen 67a, 67b auf, die offen an der Stirnfläche 17* münden, vergleichbar mit der Stirnfläche 17 bzw. 17', die jeweils dem Myokard zugewandt orientiert ist. Die nutförmigen Ausnehmungen 67a, 67b, sind im gezeigten Ausführungsbeispiel jeweils als kreisförmige und konzentrisch um die Fügekontur 8* angeordnete Rillen mit vorzugsweise rechteckförmigen Rillenquerschnitten ausgebildet. Die radial äußere Ausnehmung 67a sowie die radial innenliegende Ausnehmung 67a sind über Verbindungskanäle 68 fluidisch miteinander verbunden. Die Verbindungskanäle 68 sind hierzu jeweils in Art von Verbindungshülsen ausgebildet und durchragen die radial zwischen den nutförmigen Ausnehmungen 67a angeordnete nutförmige Ausnehmung 67b fluiddicht. In dem dargestellten Ausführungsbeispiel sind vier in Umfangsrichtung der kreisförmigen Ausnehmungen 67a, 67b gleichverteilt angeordnete Verbindungskanäle 68 vorgesehen. Durch den peripheren Umfangsrand des Kragens 10* ist eine Zugangsöffnung 69 zum Lumen der radial äußeren Ausnehmung 67a eingebracht, die vorzugsweise zum fluiddichten Anschluss an eine Unterdruckquelle ausgebildet ist, über die Unterdruck innerhalb der radial äußeren und inneren Ausnehmung 67a appliziert werden kann. Denkbar wäre auch ein Anschluss an eine Medienquelle zur Versorgung der radial äußeren und inneren Ausnehmungen 67a mit einem Medium, bspw. bioverträglicher Klebstoff.

Ferner sind durch die rückseitige Stirnseite 11* des modifizierten Kragens 10* zwei Durchgangsöffnungen 70 eingebracht, die einen fluidischen Zugang zur mittig angeordneten Ausnehmung 67b schaffen. Es sei an dieser Stelle erwähnt, dass die die radial mittig angeordnete, rillenförmige Ausnehmung 67b lokal traversierenden Verbindungskanäle 68 einen kleineren Außendurchmesser besitzen als die Tiefe der nutförmigen Ausnehmung 67b. Auf diese Weise kann sich bei Einspeisen eines Mediums in das Lumen der radial mittig angeordneten Ausnehmung 67b das Medium gleichmäßig verteilen und das Lumen vollständig ausfüllen. Zum Zwecke des Einspeisens bzw. Befüllen des Lumens wird ein Medium durch eine der beiden Durchgangsöffnungen eingespeist. Tritt das eingespeiste Medium durch die andere Durchgangsöffnung aus, so ist das Lumen vollständig gefüllt.

Unter dem Begriff "Lumen", der auch vorstehend in Zusammenhang mit den Ausnehmungen 67a verwendet wurde, sei das jeweils von einer kreisförmigen, nutförmigen Ausnehmung 67a, 67b umschlossene Volumen verstanden, das zusätzlich in Richtung der jeweils einseitig offenen Seite durch Anlage der Stirnfläche 17* des Kragens 10* an einer Begrenzungsfläche, bspw. in Form der Myokardaußenwand, begrenzt wird.

Die dargestellte Modifikation des Kragens 10* ermöglicht eine alternative Fixationsmöglichkeit des Befestigungsmoduls 2* an der Myokardwand, indem nach dem Positionieren des Befestigungsmoduls am Myokard ein Unterdruck bzw. Sog, vermittels einer Unterdruckquelle 71, siehe Fig. 9c, über die Durchgangsöffnung 69 längs der radial inneren und äußeren Ausnehmungen 66a temporär appliziert wird. Hierdurch saugt sich die Stirnfläche 17* gesamtheitlich an die Myokardwand an, wodurch insbesondere die mittlere Ausnehmung 67b durch Sogbedingtes fluiddichtes Anpressen der die mittlere nutförmige Ausnehmung jeweils radial beidseitig begrenzenden Nutwände mit der Myokardwand ein fluiddichtes Lumen einschließt. Sollte der Arzt eine Fehlpositionierung des Befestigungsmoduls feststellen, können Nachjustierungen vorgenommen werden, indem der Unterdruck zum Zwecke einer Neu- oder Nachpositionierung des Befestigungsmoduls am Myokard kurzzeitig abgebaut wird. Optional weist der Kragen 10* am Umfangsrand angebrachte Befestigungsstrukturen 16* auf, vergleiche die vorstehende Beschreibung zu den Figuren 3 und 8.

Nachfolgend erfolgt eine Kleberinjektion durch eine der beiden Durchgangsöffnungen 70 in das Lumen der mittleren, nutförmigen Ausnehmung 67b, vermittels eine Medienquelle 72, die zur dosierten Abgabe von biokompatiblen Kleber geeignet ausgebildet ist, siehe Fig. 9c. Der biokompatible Kleber verteilt sich gleichmäßig innerhalb der nutförmigen Ausnehmung 67b und bildet eine ringförmige Haftfläche aus, die die lochartig ausgebildete Läsion innerhalb der Myokardwand, durch die das Befestigungsmodul hindurchragt, luftdicht umschließt. Eine vollständige Befüllung des Lumens mit Kleber ist gegeben, wenn der Kleber aus der längs der nutförmigen Ausnehmung 67b zur Kleberinjektion gegenüberliegenden Durchgangsöffnung 70 wieder austritt.

Die vorstehend beschriebene Ausführungsform vereinfacht somit nicht nur die Befestigung des Befestigungsmoduls an der Myokardwand, sondern stellt auch sicher, dass dies nach Aussen sicher luftdicht abgeschlossen ist. Auf diese Weise können Luftembolien ausgebschlossen werden.
Die Modularität des lösungsgemäß ausgebildeten implantierbaren Fluidpumpensystems ermöglicht nicht nur einen separaten Austausch einzelner Komponenten, sondern bietet darüber hinaus auch die Möglichkeit einer individuellen Zusammenstellung der einzelnen Komponenten in Form und Größe an die jeweiligen patientenindividuellen anatomischen Bedürfnisse und Verhältnisse. So können unterschiedliche in Form und Größe aber aufeinander abgestimmte Einzelkomponenten gleichsam eines Baukastensortimentes bevorratet werden, um so dem jeweils behandelnden Arzt individuelle Entscheidungsmöglichkeiten bei der Zusammenstellung des zu implantierenden Fluidpumpensystems zu bieten.

Selbstverständlich können Form und Größe der einzelnen Komponenten sowie bspw. die relative Anordnung des ersten und zweiten Fluidkanals 5, 6 bzw. 5', 6' beim Intrakardialmodul 1, 1' abweichend von den illustrierten, konkreten Ausführungsformen, ausgebildet werden. Wesentlich ist, dass ein pumpenangetriebener Blutfluss aus der Herzkammer über das Pumpenmodul und unmittelbar oder mittelbar über den tubusförmigen Fortsatz in die Aorta bewerkstelligt werden kann. Gleichwohl ist es möglich, das implantierbare Fluidpumpensystem auch rechtsventrikulär zu implantieren, wobei in diesem Falle das offene Tubenende 51, 51' oder ein mit dem offenen Tubenende 51, 51' verbundener weiterführender Hohlkanal in der Pulmonalarterie zu liegen kommt.

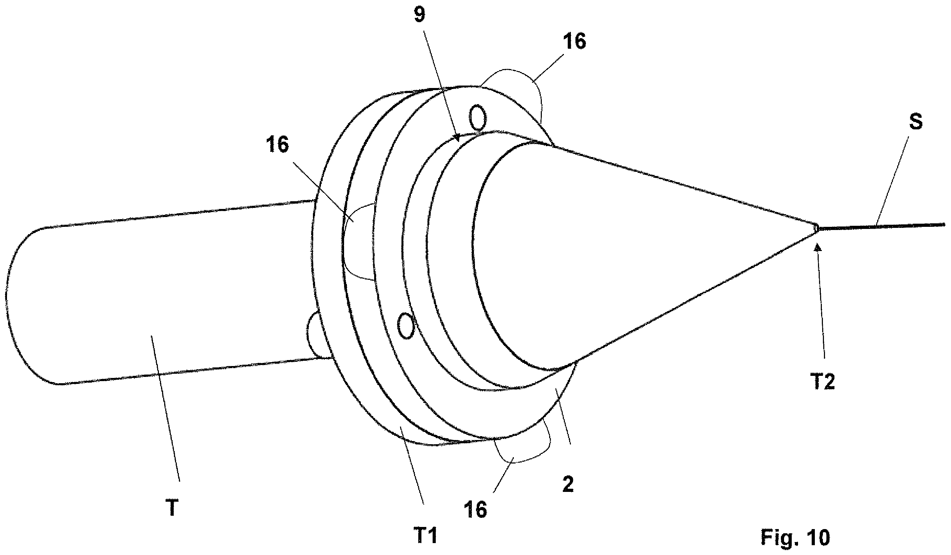

Das lösungsgemäße Fluidpumpensystem ermöglicht die Anwendung der minimalinvasiven Operationstechnik, bei der nach einer apikalen Punktion des Myokards ein Seldinger Draht S intrakardial in der Aorta platziert wird. Mit Hilfe eines in Figur 10 illustrierten Trokars T, auf dessen Trokarschulter T1 das Befestigungsmodul 2, des vorstehend beschriebenen ersten Ausführungsbeispiels gemäß der Figuren 1 bis 3 aufgesetzt ist, wird die konisch zulaufende Trokarspitze T2 mit Hilfe des Seldinger Drahtes S apikal am Myokard platziert. Durch schonendes Vorschieben der Trokarspitze T2 in das Myokard wird dieses radial seitlich aufgeweitet, bis das Myokard an der radialen Außenseite des tubusförmigen Abschnittes 9 anliegt. Anschließend erfolgt eine chirurgische Befestigung des Befestigungsmoduls 2 am Myokard. Der Trokar T wird proximalseitig entfernt und durch das Intrakardialmodul, an dem bereits das Pumpenmodul mit Hilfe des Haltemoduls vormontiert ist, ersetzt.

Im Falle des vorstehend beschriebenen zweiten Ausführungsbeispiels gemäß der Figuren 6 bis 8 ist ein Trokar T zur Einführung des Haltemoduls 2' durch die Myokardwand nicht zwingend erforderlich, zumal aufgrund der schlanken Bauform des Intrakardialmoduls 1' das Haltemodul 2' und das Intrakardialmodul 1' gemeinsam, d.h. das Intrakardialmodul 1' ist im Haltemodul 2' angeordnet, implantiert werden können. Vorsorge gilt es zu treffen, dass während der Implantation die intrakardialen Fluidkanalöffnungen 51' und 61' verschlossen sind. Ein Verschließen dieser Fluidkanalöffnungen 51' und 61' kann durch geeignete Abdeckkulissenflächen realisiert werden, die durch Rotation des Intrakardialmoduls 1' innerhalb des Haltemoduls 2' die betreffenden Fluidkanalöffnungen weitgehend fluiddicht abdecken.

Die feste Verbindung zwischen dem Intrakardialmodul und dem am Myokard befestigten Befestigungsmodul erfolgt mit Hilfe der vorstehend erläuterten Schraubverbindungen. Sollten nachträgliche Wartungs- oder Austauschmaßnahmen erforderlich sein, so insbesondere ein Austausch des Pumpenmoduls, so bedarf es keiner Irritation des Myokards, zumal das Pumpenmodul extrakardial angeordnet und entsprechend minimalinvasiv ersetzbar ist.

### Bezugszeichenliste

- 1,1': Intrakardialmodul
- 11a, 11a': Intrakardialer Abschnitt
- 12a, 12a': Medialer Abschnitt
- 13a, 13a: Extrakardialer Abschnitt
- 2, 2': Befestigungsmodul
- 3: Pumpenmodul
- 4, 4': Haltemodul
- 5, 5': erster Fluidkanal
- 6, 6': zweiter Fluidkanal
- 7, 7': Fügekontur
- 8, 8': Fügekontur
- 9, 9", 9*: Tubusförmiger Abschnitt
- 9': Konischer Abschnitt
- 9"': Konischer Abschnitt
- 10, 10', 10*: Kragen
- 11, 11', 11*: Stirnseite
- 12, 12': Abstützfläche
- 13: Ausnehmung
- 14: Schraube
- 15: Schraubenöffnung
- 16: Befestigungsstruktur
- 17, 17', 17*: Stirnfläche
- 31: Rohrabschnitt
- 311: Steckstruktur
- 312: Stutzen
- 32: Rohrabschnitt
- 321: Steckstruktur
- 322: Verbindungsstutzen
- 41: Durchtrittsöffnung
- 42: Durchtrittsöffnung
- 43: Befestigungsschraube
- 44: Schraubenöffnung
- 45: Innengewinde
- 46: Befestigungsöffnung
- 47: Befestigungsschraube
- 51, 51': Intrakardiale Fluidkanalöffnung
- 52, 52': Tubenartiger Fortsatz
- 53, 53': Verbindungsstruktur
- 54, 54': Extrakardiale Fluidkanalöffnung
- 55: Stufenartige Einsteckkontur
- 61, 61': Intrakardiale Fluidkanalöffnung
- 62, 62': Extrakardiale Fluidkanalöffnung
- 63: Stufenartige Einsteckkontur
- 64: Aussenrohr
- 65: Übergangshülse
- 66: Durchgangsöffnung
- 67a: Nutförmige Ausnehmung
- 67b: Nutförmige Ausnehmung
- 68: Verbindungskanal
- 69: Durchgangsöffnung
- 70: Durchgangsöffnung
- 71: Unterdruckquelle
- 72: Medienquelle
- T: Trokar
- T1: Trokarschulter
- T2: Trokarspitze
- S: Seldingerdraht

## Patentansprüche

1. Implantierbares Fluidpumpensystem zur Strömungsunterstützung oder
- initiierung innerhalb eines Fluid-durchströmten Hohlorgans, insbesondere des Herzens, mit
- einem Intrakardialmodul (1), das zwei separate Fluidkanäle umfasst, die jeweils wenigstens eine intrakardiale Fluidkanalöffnung und wenigstens eine dieser gegenüberliegende, extrakardiale Fluidkanalöffnung besitzen,
- einem Befestigungsmodul (2), das eine Fügekontur (8) zum fluiddichten Fügen an das Intrakardialmodul sowie eine Befestigungsstruktur (16) zur intrakorporalen Befestigung an das Hohlorgan vorsieht, sowie
- einem extrakardialen Pumpenmodul (3), das mittelbar lösbar fest an das Intrakardialmodul anbringbar und fluiddicht an die extrakardialen Fluidkanalöffnungen zur Herstellung einer fluiddichten Verbindung beider Fluidkanäle anschließbar ist, wobei das Pumpenmodul in einem extrakorporalen Haltemodul (4) lösbar fest fixiert ist, das vermittels einer lösbaren Fügeverbindung an den extrakardialen Abschnitt des Intrakardialmoduls fluiddicht fixiert ist.

2. Implantierbares Fluidpumpensystem nach Anspruch 1,
**dadurch gekennzeichnet, dass** das Intrakardialmodul in einer axialen Abfolge einen intrakardialen, einen medialen und einen extrakardialen Abschnitt aufweist,
dass der intrakardiale Abschnitt sowohl einen tubenartigen Fortsatz mit einem offenen distalen Tubenende, der einen der beiden Fluidkanäle umfasst, sowie lateral zum oder radial um den tubenartigen Fortsatz angeordnet die wenigstens eine intrakardiale Fluidkanalöffnung des anderen Fluidkanals aufweist,
dass der mediale Abschnitt eine peripher umlaufende Fügekontur besitzt, die gegenkonturiert zur Fügekontur des Befestigungsmodul ausgebildet ist, und
dass am extrakardialen Abschnitt die extrakardialen, offenen Fluidkanalöffnungen beider Fluidkanäle offen münden.

3. Implantierbares Fluidpumpensystem nach Anspruch 2,
**dadurch gekennzeichnet, dass** die den medialen Abschnitt des Intrakardialmoduls peripher umlaufende Fügekontur geradzylinderförmig, konisch oder prismatisch ausgebildet ist, und
dass das Befestigungsmodul ringartig mit einer radial innenliegenden Öffnung ausgebildet ist, die von der Fügekontur des Befestigungsmoduls peripher umfasst ist, die geradzylinderförmig, konisch oder prismatisch ist.

4. Implantierbares Fluidpumpensystem nach einem der Ansprüche 1 bis 3,
**dadurch gekennzeichnet, dass** das Befestigungsmodul eine propfenartige Raumform besitzt mit einem tubusförmigen Abschnitt, der radial innenseitig wenigstens teilweise die Fügekontur umfasst und an den sich einseitig ein den tubusförmigen Abschnitt radial überragender Kragen anschließt.

5. Implantierbares Fluidpumpensystem nach Anspruch 4,
**dadurch gekennzeichnet, dass** der Kragen eine dem tubusförmigen Abschnitt abgewandte Stirnfläche besitzt, die in Form und Größe einer am extrakardialen Abschnitt des Intrakardialmoduls vorgesehenen Abstützfläche entspricht, an der die Stirnfläche im einem gefügten Zustand zwischen Intrakardialmodul und Befestigungsmodul bündig anliegt.

6. Implantierbares Fluidpumpensystem nach einem der Ansprüche 1 bis 5,
**dadurch gekennzeichnet, dass** das Pumpenmodul ein U-förmiges Rohrleitungssystem umfasst, längs dem eine motorisch angetriebene Fluidförderpumpe eingebracht ist, und
dass das U-förmige Rohrleitungssystem zwei offen endende Rohrleitungsstutzen vorsieht, die im Wege einer fluiddichten Steckverbindung mit den extrakardialen Fluidkanalöffnungen der vom Intrakardialmodul umfassten Fluidkanäle fluiddicht und lösbar fest verbunden sind.

7. Implantierbares Fluidpumpensystem nach Anspruch 6,
**dadurch gekennzeichnet, dass** das U-förmige Rohrleitungssystem des Pumpenmoduls zwei trennbare Rohrleitungsabschnitte aufweist, längs dessen einen Rohrleitungsabschnitt die motorisch angetriebene Förderpumpe integriert ist.

8. Implantierbares Fluidpumpensystem nach Anspruch 2 und 7,
**dadurch gekennzeichnet, dass** der offen endende Rohrleitungsstutzen des einen Rohrleitungsabschnittes, längs dem die motorisch angetriebene Förderpumpe integriert ist, mit dem extrakardialen Fluidkanalende des anderen Fluidkanals fluiddicht und lösbar fest verbunden ist.

9. Implantierbares Fluidpumpensystem nach einem der Ansprüche 4 bis 8,
**dadurch gekennzeichnet, dass** der Kragen eine dem tubusförmigen Abschnitt zugewandte ringförmige Stirnfläche besitzt, an der mittel- oder unmittelbar ein die ringförmige Stirnfläche radial zumindest teilweise überragendes und als Befestigungsstruktur dienendes Flächenelement angebracht ist, das derart ausgebildet ist, dass das Flächenelement mit einer chirurgischen Nadel durchstochen werden kann zu Zwecken einer mittels eines chirurgischen Fadens am Hohlorgan realisierbaren Fixierung des implantierbaren Fluidpumpensystems.

10. Implantierbares Fluidpumpensystem nach einem der Ansprüche 2 bis 9,
**dadurch gekennzeichnet, dass** im Bereich des distalen Tubenendes des tubenartigen Fortsatzes eine Verbindungsstruktur angebracht ist zum fluiddichten Anbringen eines den tubenartigen Fortsatzes verlängernden künstlichen Hohlkanalabschnittes.

11. Implantierbares Fluidpumpensystem nach Anspruch 10,
**dadurch gekennzeichnet, dass** die Verbindungsstruktur eine Steck- oder Schraubgewindekontur vorsieht.

12. Implantierbares Fluidpumpensystem nach einem der Ansprüche 1 bis 11,
**dadurch gekennzeichnet, dass** die beiden Fluidkanäle lateral versetzt oder koaxial zueinander innerhalb des Intrakardialmoduls angeordnet sind.

13. Implantierbares Fluidpumpensystem nach einem der Ansprüche 4 bis 8,
**dadurch gekennzeichnet, dass** der Kragen eine dem tubusförmigen Abschnitt zugewandte ringförmige Stirnfläche besitzt,
dass der Kragen wenigstens zwei zur Stirnfläche einseitig offen ausgebildete und getrennt voneinander ausgebildete, nutförmige Ausnehmungen besitzt, und
dass die Ausnehmungen separat voneinander jeweils mit wenigstens einer den Kragen lokal durchdringenden Durchgangsöffnung verbunden sind.

14. Implantierbares Fluidpumpensystem nach Anspruch 13,
**dadurch gekennzeichnet, dass** eine Unterdruckquelle fluiddicht über eine Durchgangsöffnung mit wenigstens einer Ausnehmung und eine Medienquelle fluiddicht über eine andere Durchgangsöffnung mit wenigstens einer anderen Ausnehmung verbunden sind.

15. Implantierbares Fluidpumpensystem nach Anspruch 13 oder 14,
**dadurch gekennzeichnet, dass** wenigstens drei an der ringförmigen Stirnfläche, konzentrisch zueinander angeordnete, nutförmige Ausnehmungen offen einseitig münden,
dass wenigstens eine der drei Ausnehmungen von einer radial äußeren und einer radial inneren Ausnehmung umgeben ist,
dass die wenigstens eine nutförmige Ausnehmung die zwischen der radial Äußeren und radial Inneren angeordnet ist über wenigstens eine den Kragen mittel- oder unmittelbar lokal durchdringende Durchgangsöffnung mit einer Medienquelle oder Unterdruckquelle verbindbar ist,
dass die radial äußere und radial innere Ausnehmung über wenigstens einen Verbindungskanal fluidisch miteinander kommunizieren und dass die radial äußere und/oder radial innere Ausnehmung über wenigstens eine den Kragen mittel- oder unmittelbar lokal durchdringende Durchgangsöffnung mit einer Medienquelle oder Unterdruckquelle verbindbar ist/sind.

## Claims

1. Implantable fluid pump system for supporting or initiating flow inside a hollow organ through which fluid circulates, in particular the heart, with
- an intracardiac module (1), which comprises two separate fluid channels, each of which possesses at least one intracardiac fluid channel opening and, located opposite the latter, at least one extracardiac fluid channel opening,
- a fastening module (2), which provides a join contour (8) for purposes of joining to the intracardiac module in a fluid-tight manner, and provides a fastening structure (16) for purposes of intracorporeal fastening to the hollow organ, and
- an extracardiac pump module (3), which can be indirectly securely mounted in a releasable manner onto the intracardiac module, and can be connected in a fluid-tight manner to the extracardiac fluid channel openings so as to produce a fluid-tight connection of both fluid channels, wherein
the pump module is securely fixed in a releasable manner in an extracorporeal retaining module (4), which is fixed in a fluid-tight manner by means of a releasable joint connection onto the extracardiac section of the intracardiac module.

2. Implantable fluid pump system according to Claim 1,
**characterised in that**,
the intracardiac module in an axial sequence has an intracardiac, a medial and an extracardiac section,
**in that** the intracardiac section has both a tubular extension with an open distal tube end, which surrounds one of the two fluid channels, and, arranged laterally spaced from, or radially around, the tubular extension, the at least one intracardiac fluid channel opening of the other fluid channel,
**in that** the medial section possesses a peripherally surrounding join contour, which is designed as a contour matching the join contour of the fastening module,
and **in that**, the extracardiac, open fluid channel openings of both fluid channels open out into the extracardiac section.

3. Implantable fluid pump system according to Claim 2,
**characterised in that**,
the join contour peripherally surrounding the medial section of the intracardiac module is of a straight cylindrical, conical, or prismatic form,
and **in that**, the fastening module is designed in the form of a ring with a radially inner opening, which is peripherally surrounded by the join contour of the fastening module, which is of a straight cylindrical, conical or prismatic form.

4. Implantable fluid pump system according to one of the Claims 1 to 3,
**characterised in that**,
the fastening module possesses a plug-type spatial form with a tubus-form section, which on the radially inner side at least partially surrounds the join contour, and onto which a collar projecting radially beyond the tubus-form section connects on one side.

5. Implantable fluid pump system according to Claim 4,
**characterised in that**,
the collar possesses an end face facing away from the tubus-form section, which corresponds in shape and size to a supporting surface provided on the extracardiac section of the intracardiac module, on which in a joined state the end face of the fastening module fits flush between the intracardiac module and the fastening module.

6. Implantable fluid pump system according to one of the Claims 1 to 5,
**characterised in that**,
the pump module comprises a U-shaped piping system, along which a motor-driven fluid feed pump is introduced,
and **in that**, the U-shaped piping system has two open-ended pipe stubs, which are securely connected in a fluid-tight and releasable manner by way of a fluid-tight plug-in connection to the extracardiac fluid channel openings of the fluid channels surrounded by the intracardiac module.

7. Implantable fluid pump system according to Claim 6,
**characterised in that**,
the U-shaped piping system of the pump module has two separable pipe sections; the motor-driven pump is integrated along one of these pipe sections.

8. Implantable fluid pump system according to Claims 2 and 7,
**characterised in that**,
the open-ended pipe stub of the one pipe section, along which the motor-driven feed pump is integrated, is securely connected in a fluid-tight and releasable manner to the extracardiac fluid channel end of the other fluid channel.

9. Implantable fluid pump system according to one of the Claims 4 to 8,
**characterised in that**,
the collar possesses an annular end face facing towards the tubus-form section, onto which is mounted directly or indirectly a surface element at least partially projecting radially beyond the annular end face and serving as a fastening structure; which surface element is designed such that it can be pierced with a surgical needle for purposes of an attachment of the implantable fluid pump system onto the hollow organ that can be implemented by means of a surgical thread.

10. Implantable fluid pump system according to one of the Claims 2 to 9,
**characterised in that**,
in the region of the distal tube end of the tubular extension, a connecting structure is mounted for the fluid-tight fitting of an artificial hollow channel section lengthening the tubular extension.

11. Implantable fluid pump system according to Claim 10,
**characterised in that**,
the connecting structure provides a plug-in or a screw thread contour.

12. Implantable fluid pump system according to one of the Claims 1 to 11,
**characterised in that**,
the two fluid channels are arranged either laterally offset from, or coaxially with, one another within the intracardiac module.

13. Implantable fluid pump system according to one of the Claims 4 to 8,
**characterised in that**,
the collar possesses an annular end face facing towards the tubus-form section,
**in that** the collar possesses at least two groove-shaped recesses designed to be open to the end face on one side, and separate from one another,
and **in that**, in each case the recesses are connected,
separately from one another, with at least one passageway locally penetrating the collar.

14. Implantable fluid pump system according to Claim 13,
**characterised in that**,
a reduced pressure source is connected in a fluid-tight manner via a passageway with at least one recess, and a media source is connected in a fluid-tight manner via another passageway with at least one other recess.

15. The implantable fluid pump system in accordance with claim 13 or 14,
**characterised in that**,
at least three groove-shaped recesses, arranged concentrically with one another, open out on one side onto the annular end face,
**in that** at least one of the three recesses is surrounded by a radially outer and a radially inner recess,
**in that** the at least one groove-shaped recess, which is arranged between the radially outer and the radially inner recesses, can be connected via at least one passageway that locally penetrates the collar directly or indirectly, to a media source or a reduced pressure source,
**in that** the radially outer and radially inner recesses communicate with each other in terms of fluid flow via at least one connecting channel,
and **in that**, the radially outer and/or the radially inner recess can be connected via at least one passageway, which locally penetrates the collar directly or indirectly, to a media source or a reduced pressure source.

## Revendications

1. Système implantable de pompe à fluide, destiné à assister ou à initier une circulation à l'intérieur d'un organe creux à travers lequel s'écoule un fluide, notamment du cœur,
- pourvu d'un module intracardiaque (1), lequel comprend deux canaux à fluide séparés, dont chacun détient au moins un orifice de canal à fluide intracardiaque et au moins un orifice de canal à fluide extracardiaque, au vis-à-vis de de celui-ci,
- pourvu d'un module de fixation (2), lequel prévoit un contour de raccordement (8) pour le raccordement étanche aux fluides sur le module intracardiaque, ainsi qu'une structure de fixation (16), pour la fixation intracorporelle sur l'organe creux, ainsi que
- pourvu d'un module de pompe (3) extracardiaque, qui est susceptible d'être monté fixement, de manière indirectement amovible sur le module intracardiaque et d'être raccordé de manière étanche aux fluides sur les orifices de canal à fluide extracardiaques, aux fins d'établir une liaison étanche aux fluides entre les deux canaux à fluide,
le module de pompe extracardiaque étant fixé de manière fixe amovible dans un module de maintien (4), qui est fixé de manière étanche aux fluides au moyen d'un aboutement amovible sur le segment extracardiaque du module intracardiaque.

2. Système implantable de pompe à fluide selon la revendication 1,
**caractérisé en ce que** le module intracardiaque comporte dans une succession axiale un segment intracardiaque, un segment médian et un segment extracardiaque,
**en ce que** le segment intracardiaque comprend aussi bien un prolongement tubulaire pourvu d'une extrémité de tube distale ouverte, qui comprend l'un des deux canaux à fluide, ainsi qu'en placement latéral par rapport au prolongement tubulaire ou en direction radiale autour de celui-ci l'au moins un orifice de canal à fluide de l'autre canal à fluide,
**en ce que** le segment médian détient un contour de raccordement périphérique, qui est conçu avec un contour inverse à celui du contour de raccordement du module de fixation, et
**en ce que** sur le segment extracardiaque débouchent ouvertement les orifices de canal à fluide extracardiaques ouverts des deux canaux à fluide.

3. Système implantable de pompe à fluide selon la revendication 2,
**caractérisé en ce que** le contour de raccordement périphérique du segment médian du module intracardiaque est conçu en forme de cylindre droit, de forme conique ou prismatique, et
**en ce que** le module de fixation est conçu de forme annulaire, avec un orifice interne en direction radiale qui est entouré de manière périphérique par le contour de raccordement du module de fixation qui est en forme de cylindre droit, de forme conique ou prismatique.

4. Système implantable de pompe à fluide selon l'une quelconque des revendications 1 à 3,
**caractérisé en ce que** le module de fixation détient une forme spatiale de tampon, avec un segment tubulaire, qui entoure en direction radiale sur le côté interne au moins en partie le contour de raccordement et sur lequel se raccorde d'un côté une collerette saillant en direction radiale par-dessus le segment tubulaire.

5. Système implantable de pompe à fluide selon la revendication 4,
**caractérisé en ce que** la collerette détient une face frontale au vis-à-vis du segment tubulaire, qui correspond par sa forme et sa taille à une surface d'appui prévue sur le segment extracardiaque du module intracardiaque, sur laquelle dans une position raccordée du module intracardiaque et du module de fixation, la face frontale est appuyée à fleur.

6. Système implantable de pompe à fluide selon l'une quelconque des revendications 1 à 5, **caractérisé en ce que** le module de pompe extracardiaque comprend un système de tuyauterie en forme de U, le long duquel est introduite une pompe de transfert de fluide entraînée par moteur, et
**en ce que** le système de tuyauterie en forme de U prévoit deux tubulures de tuyauterie avec des extrémités ouvertes, qui par voie d'un enfichage étanche aux fluides sont reliées de manière étanche aux fluides et fixe amovible avec les orifices de canal à fluide extracardiaques des canaux à fluide englobés par le module intracardiaque.

7. Système implantable de pompe à fluide selon la revendication 6,
**caractérisé en ce que** le système de tuyauterie en forme de U du module de pompe comporte deux tronçons de tuyauterie séparables, le long de l'un des tronçons de tuyauterie étant intégrée la pompe de transfert entraînée par moteur.

8. Système implantable de pompe à fluide selon la revendication 2 et 7,
**caractérisé en ce que** la tubulure de tuyauterie à extrémités ouvertes du tronçon de tuyauterie le long duquel est intégrée la pompe de transfert entraînée par moteur est reliée de manière étanche aux fluides et fixe amovible avec l'extrémité de canal à fluide extracardiaque de l'autre canal à fluide.

9. Système implantable de pompe à fluide selon l'une quelconque des revendications 4 à 8, **caractérisé en ce que** la collerette détient une face frontale annulaire qui est dirigée vers le segment tubulaire, sur laquelle est monté un élément plan saillant au moins partiellement par-dessus la surface frontale annulaire et faisant office de structure de fixation, qui est conçu de telle sorte que l'élément plan puisse être percé à l'aide d'une aiguille chirurgicale, aux fins de la fixation du système implantable de pompe à fluide sur l'organe creux, réalisable au moyen d'un fil chirurgical.

10. Système implantable de pompe à fluide selon l'une quelconque des revendications 2 à 9, **caractérisé en ce que** dans la zone de l'extrémité distale de tube du prolongement tubulaire est montée une structure de liaison, destinée au montage étanche aux fluides d'un tronçon de canal creux artificiel, qui rallonge le prolongement tubulaire.

11. Système implantable de pompe à fluide selon la revendication 10,
**caractérisé en ce que** la structure de liaison prévoit un contour d'enfichage ou un contour fileté.

12. Système implantable de pompe à fluide selon l'une quelconque des revendications 1 à 11,
**caractérisé en ce que** les deux canaux à fluide sont placés avec un décalage latéral ou de manière coaxiale l'un par rapport à l'autre à l'intérieur du module intracardiaque.

13. Système implantable de pompe à fluide selon l'une quelconque des revendications 4 à 8, **caractérisé en ce que** la collerette détient une surface frontale annulaire qui est dirigée vers le segment tubulaire, **en ce que** la collerette détient au moins deux évidements en forme de rainures, conçus en étant ouverts d'un côté et conçus séparément l'un de l'autre, et
**en ce que** les évidements sont reliés séparément l'un de l'autre, chacun avec au moins un orifice de passage traversant localement la collerette.

14. Système implantable de pompe à fluide selon la revendication 13,
**caractérisé en ce qu'**une source de dépression est reliée de manière étanche aux fluides par l'intermédiaire d'un orifice de passage avec au moins un évidement et **en ce qu'**une source de fluide est reliée de manière étanche aux fluides par l'intermédiaire d'un autre orifice de passage avec au moins un autre évidement.

15. Système implantable de pompe à fluide selon la revendication 13 ou 14,
**caractérisé en ce qu'**au moins trois évidements placés de manière concentrique l'un par rapport à l'autre sur la face frontale annulaire débouchent de manière ouverte par un côté,
**en ce qu'**au moins l'un des trois évidements est entouré d'un évidement extérieur en direction radiale et d'un évidement intérieur en direction radiale,
**en ce qu'**au moins l'un évidement en forme de rainure qui est placé entre l'évidement extérieur en direction radiale et l'évidement intérieur en direction radiale est susceptible d'être relié par l'intermédiaire d'au moins un orifice de passage traversant localement, indirectement ou directement la collerette avec une source de fluide ou une source de dépression,
**en ce que** l'évidement extérieur en direction radiale et l'évidement intérieur en direction radiale communiquent fluidiquement l'un avec l'autre par l'intermédiaire d'un canal de liaison et **en ce que** l'évidement extérieur en direction radiale et/ou l'évidement intérieur en direction radiale est/sont susceptible(s) d'être reliés par l'intermédiaire d'au moins un orifice de passage traversant localement, indirectement ou directement la collerette avec une source de fluide ou une source de dépression.
